# EUROPEAN PATENT APPLICATION

(11) **EP 1 038 538 A1**
(43) Date of publication of application: **27.09.2000**
(21) Application number: 99200865.6
(22) Date of filing: 19.03.1999
(51) Int. Cl.: A61L 27/00

(54) **Muscle tissue engineering**

(71) Applicant: IsoTis B.V., 3723 MB Bilthoven (NL)
(72) Inventor: The designation of the inventor has not yet been filed
(74) Representative: Ottevangers, Sietse Ulbe

(57) **Abstract**

The invention relates to the use of a matrix of a copolymer of a polyalkylene glycol and an aromatic polyester as a scaffold for tissue engineering muscle.

## Description

The invention relates to a scaffold for tissue engineering muscle tissue.

Human muscle tissue has a very limited capacity for regeneration. Cardiac muscle tissue even lacks this capacity completely. Consequently, patients suffering from muscle injuries or muscle disorders need quite a long time before they are recovered, if recovery is at all possible.

In the past, it has been postulated that it should be possible to transplant skeletal or cardiac muscle tissue. This would specifically be desirable for patients undergoing plastic or reconstructive surgery, or suffering from muscle dystrophy or heart disease (i.e. cases where recovery of propagation of electrophysiological pulse around a non-functional area is required). Currently, transplantation of autologous or allogenic tissue is employed. However, a drawback of these methods is foremost that the required tissue is extremely scarce, and thus often unavailable when needed. Furthermore, the use of allogenic tissue is associated with a significant risk of infections and the need of powerful immunosuppressant drugs.

In US patent 5,759,830, it has been proposed that a three-dimensional fibrous scaffold may be used for culturing cells on its surface for producing vascularized tissue in vivo. In accordance with this approach, autologous cells are cultured on a scaffold in vitro. The scaffold having cultured autologous cells attached thereto can subsequently be implanted in a patient having a specific disorder in order to achieve tissue regeneration. After implantation, the attached, pre-cultured cells regenerate the missing or defective tissue, while the scaffold is degraded. In the US patent is stated that these scaffolds can be used for various cell types, among which blood vessel cells, various organ tissue cells, epithelial cells, nerve cells, and muscle cells.

It is indicated that a key point in the configuration of the scaffolds is, that diffusion is enabled to its utmost extent. As the scaffold with attached cells is implanted in non-vascularized form, it is essential that nutrition can be supplied through diffusion. For this reason, it is stated that the scaffolds must have sufficient surface area and exposure to nutrients such that cellular growth and differentiation can occur prior to the ingrowth of blood vessels following implantation. In fact, the only configuration of the scaffolds that is believed to be suitable in accordance with the above US patent, is a fibrous configuration. The open space in between the fibers is believed to be essential in facilitating diffusion to a sufficient degree.

The materials used for making the scaffold described in the US patent are bioabsorbable, biodegradable, synthetic polymers. The only examples mentioned of suitable polymers are polyanhydrides, polyorthoesters, and polyglycolic acids.

The polymers proposed for use as a scaffold material in US-A-5,759,830 have been found to not have sufficient elastomeric properties to be used as a scaffold for muscle tissue. They do not, or to an insufficient extent, allow for cultivation comprising dynamic or static elongation. Further, they do not mimic the elastomeric properties of the target tissue and do not have the capacity to contribute to the biomechanical properties of the implant in vivo. Also, these materials show a rather fast biodegradation, which is associated with an increased risk of inflammatory response of the patient, as well as an unacceptable reduction in pH of the surroundings of the implant. Thus, regeneration is not favored, but the rather quick repair leads to scar tissue formation instead, which is undesired.

It is an object of the present invention to provide an improved scaffold for muscle tissue engineering and to thereby overcome the problems associated with the scaffolds described in the prior art. Specifically, it is desired to provide a scaffold having elastomeric properties closely mimicking the properties of the target tissue. Further, the scaffold should have an optimal biodegradation profile. It should degrade slow enough to lead to sufficient contribution to biomechanical properties after implantation and desirably to prevent formation of scar tissue, and it should degrade fast enough to be resorbed when mechanical strength is no longer needed. In addition, the scaffold must be highly prone to cell attachment, in particular of muscle cells.

Surprisingly, it has been found that the above objects are achieved by using a scaffold based on a highly specific copolymer material. This material is a copolymer of a polyalkylene glycol and an aromatic polyester. Accordingly, the invention relates to the use of a matrix of a copolymer of a polyalkylene glycol and an aromatic polyester as a scaffold for tissue engineering muscle.

It has been found that a scaffold according to the invention does not necessarily have to be fibrous in structure for enabling a sufficient degree of diffusion before in vivo vascularization has taken place. In an aqueous environment, the copolymer on which the scaffold is based forms a hydrogel. Surprisingly, this hydrogel is permeable for nutrient transport to the cells and transport of waste products from the cells. Advantageously, the material on which the present scaffold is based is biocompatible and shows a highly favorable cell attachment.

As has been mentioned, the material on which the present scaffold is based, is a copolymer of a polyalkylene glycol and an aromatic polyester. Preferably, the copolymer comprises 40-80 wt.%, more preferably 60-70 wt.% of the polyalkylene glycol, and 60-20 wt.%, more preferably 40-30 wt.% of the aromatic polyester. A preferred type of copolymers according to the invention is formed by the group of block copolymers.

Preferably, the polyalkylene glycol has a weight average molecular weight of about 150 to about 4000, more preferably of about 200 to about 1500. The aromatic polyester preferably has a molecular weight of from about 200 to about 5000, more preferably from about 250 to about 4000. The weight average molecular weight of the copolymer preferably lies between about 20,000 and 200,000, more preferably between about 50,000 and about 120,000. The weight average molecular weight may suitably be determined by gel permeation chromatography (GPC). This technique, which is known per se, may for instance be performed using tetrahydrofuran as a solvent and polystyrene as external standard.

In a preferred embodiment, the polyalkylene glycol component has units of the formula -OLO-CO-Q-CO-, wherein O represents oxygen, C represents carbon, L is a divalent organic radical remaining after removal of terminal hydroxyl groups from a poly(oxyalkylene)glycol, and Q is a divalent organic radical.

Preferred polyalkylene glycols are chosen from the group of polyethylene glycol, polypropylene glycol, and polybutylene glycol and copolymers thereof, such as poloxamers. A highly preferred polyalkylene glycol is polyethylene glycol.

The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. The polyalkylene glycol component is preferably terminated with a dicarboxylic acid residue -CO-Q-CO-, if necessary to provide a coupling to the polyester component. Group Q may be an aromatic group having the same definition as R, or may be an aliphatic group such as ethylene, propylene, butylene and the like.

The polyester component preferably has units -O-E-O-CO-R-CO-, wherein O represents oxygen, C represents carbon, E is a substituted or unsubstituted alkylene or oxydialkylene radical having from 2 to 8 carbon atoms, and R is a substituted or unsubstituted divalent aromatic radical.

In a preferred embodiment, the polyester is chosen from the group of polyethylene terephtalate, polypropylene terephtalate, and polybutylene terephtalate. A highly preferred polyester is polybutylene terephtalate.

The preparation of the copolymer will now be explained by way of example for a polyethylene glycol/polybutylene terephtalate copolymer. Based on this description, the skilled person will be able to prepare any desired copolymer within the above described class. An alternative manner for preparing polyalkylene glycol/polyester copolymers is disclosed in US-A-3,908,201.

A polyethylene glycol/polybutylene terephtalate copolymer may be synthesized from a mixture of dimethyle terephtalate, butanediol (in excess), polyethylene glycol, an antioxidant and a catalyst. The mixture is placed in a reaction vessel and heated to about 180°C, and methanol is distilled as transesterification proceeds. During the transesterification, the ester bond with methyl is replaced with an ester bond with butylene. In this step the polyethyene glycol substantially does not react. After transesterification, the temperature is raised slowly to about 245°C, and a vacuum (finally less than 0.1 mbar) is achieved. The excess butanediol is distilled and a prepolymer of butanediol terephtalate condenses with the polyethylene glycol_to form a polyethylene/polybutylene terephtalate copolymer. A terephtalate moiety connects the polyethylene glycol units to the polybutylene terephtalate units of the copolymer and thus such copolymer is sometimes also referred to as a polyethylene glycol terephtalate/polybutylene terephtalate copolymer (PEGT/PBT copolymer).

The copolymer described above may conveniently be processed into a desired shape using any known manner such as injection molding, extrusion, and so forth. The objective shape and size will depend on the envisaged application of the scaffold, in particular on the shape and size of the defect in muscle tissue which is intended to be repaired using the present scaffold.

The copolymer may either be formed into a porous or into a fibrous scaffold, thus enabling optimal diffusion of nutrients and waste products. Porous structures may be obtained by techniques known per se, such as salt leaching or sintering. Fibrous structures can *inter alia* be obtained by extrusion. Preferably, the copolymer is formed into a fibrous structure, as this type of structure more closely resembles natural muscle tissue.

A scaffold based on the above described copolymer, which is of course also encompassed by the present invention, is particularly suitable for use in tissue engineering muscle tissue. In this application, the scaffold may be implanted with or without cells attached thereto. The swelling behavior of the copolymer material allows for swell fixation of the implant into a defect in a patient, which is especially advantageous when the scaffold is used free of cells. The extent can suitably be controlled by the skilled artisan by adjusting the composition of the copolymer material.

When the scaffold in implanted without cells attached thereto, the surrounding, healthy muscle tissue will regenerate cells which grow into the scaffold, or cells may migrate from surrounding tissue into the scaffold. This process will be accompanied by vascularization. As the regeneration process proceeds, the scaffold will be slowly broken down. Thus, eventually, the copolymer material of the scaffold will have disappeared and new muscle tissue has been formed.

Preferably, the scaffold is implanted with cells attached thereto. Thus, it is preferred that the scaffold is seeded with cells prior to implantation. The cells may be any type of cells naturally occurring in muscle tissue or any type of cells capable of differentiating into such cells. Preferred cell types are muscle cells, such as cells forming smooth muscle, cells forming skeletal muscle, or heart muscle cells, stem cells, and satellite cells (stem cells of skeletal muscle tissue). It is further preferred that the cells are autologous cells, thus minimizing the chance of rejection responses in the patient treated with the present scaffold.

The seeding may be carried out in any known manner, for instance by static seeding. It is preferred, however, that the cells are seeded dynamically as has been described in co-pending European patent application 98203774.9, which is incorporated herein by reference. Although muscle cells require very careful handling and are very difficult to attach to non-natural materials, in accordance with the present invention a good attachment can be obtained.

Subsequent to the seeding process, the cells are preferably cultured in vitro, allowing for a sufficient degree of proliferation and/or differentiation of the cells. The period required for the culturing may vary broadly and range between one hour and several months, depending on the number of seeded cells and the size of the implant or scaffold.

The invention further relates to the use of the above described scaffold as a medical implant for repairing defective or missing muscle tissue.

The invention will now be elucidated by the following, non-restrictive example.

### EXAMPLE

Squares of dense 55/45(300) Polyactive films (100 µm ± 10 µm), size 1.5x1.5 cm, were washed in 95% EtOH, rinsed in PBS, air dried and steam sterilized. A murine myogenic cell line (C2C12) was cultured in the presence of said Polyactive squares until 80% confluency in Dulbecco's Modified Eagle Medium (DMEM) supplemented with 10% Fetal Calf Serum (FCS), 10% Calf Serum (CS), penicillin/streptamycin.

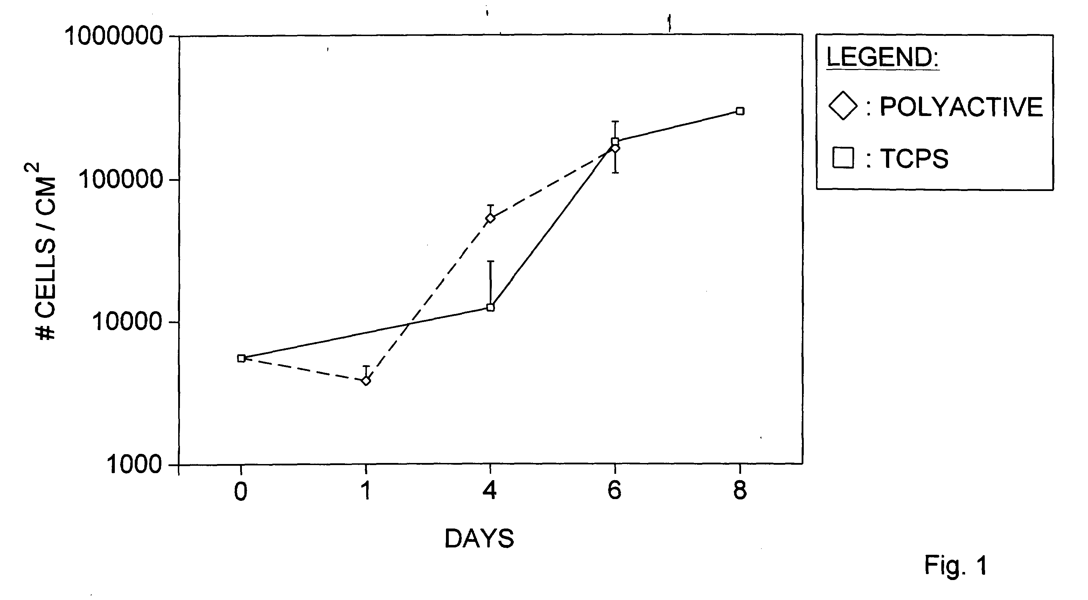

After equilibration in the culture media over night and aspiration of the media, 150 µl cell suspension was evenly distributed across the polymer surface to a final density of 5500 cell/cm² (n=3). As a control Tissue Culture Polystyrene (TCPS) was used (n=3). After 4,6 and 8 days (for control: 1,4 and 6 days) cells were trypsinized and counted. After 6 days there was no significant difference between Polyactive and TCPS indicating that Polyactive is a suitable support for myocyte proliferation. Figure 1 shows a growth curve of the cells both on TCPS and on Polyactive.

## Claims

1. The use of a matrix of a copolymer of a polyalkylene glycol and an aromatic polyester as a scaffold for tissue engineering muscle.

2. The use according to claim 1, wherein the copolymer comprises 40-80 wt.%, based on the weight of the copolymer, of the polyalkylene glycol, and 60-20 wt.%, based on the weight of the copolymer, of the aromatic polyester.

3. The use according to claim 1 or 2, wherein the polyalkylene glycol is chosen from the group of polyethylene glycol, polypropylene glycol, and polybutylene glycol.

4. The use according to claim 3, wherein the polyalkylene glycol is polyethylene glycol.

5. The use according to any of the preceding claims, wherein the aromatic polyester is chosen from the group of polethylene terephtalate, polypropylene terephtalate, and polybutylene terephtalate.

6. The use according to claim 5, wherein the aromatic polyester is polybutylene terephtalate.

7. The use according to any of the preceding claims, wherein the scaffold is provided with autologous muscle cells and/or stem cells.

8. A matrix of a copolymer of a polyalkylene glycol and an aromatic polyester to be used as a scaffold for tissue engineering muscle according to any of the preceding claims.

9. A method for repairing muscle tissue comprising implantation of a scaffold according to claim 8.

10. The use of a copolymer of a polyalkylene glycol and an aromatic polyester for the manufacture of a scaffold for implantation in a patient for repairing muscle tissue.

11. A method for manufacturing an implant for muscle tissue, wherein a matrix of a copolymer of a polyalkylene glycol and an aromatic polyester is provided with autologous muscle cells and/or stem cells by immersing the matrix in a culture medium, which culture medium comprises the cells and is kept under dynamic conditions.

12. A method according to claim 11, wherein the cells are cultured under dynamic conditions.
